# EUROPÄISCHE PATENTSCHRIFT

(11) **EP 3 663 755 B1**
(45) Veröffentlichungstag und Bekanntmachung des Hinweises auf die Patenterteilung: **06.03.2024**
(21) Anmeldenummer: 18210220.2
(22) Anmeldetag: 04.12.2018
(51) Int. Cl.: C12Q 1/02

(54) **VERFAHREN ZUR ÖKOTOXIKOLOGISCHEN TESTUNG MINDESTENS EINES WIRKSTOFFES AN EINEM STANDARDISIERTEN BIOFILM**
METHOD FOR ECOTOXICOLOGICAL TESTING OF AT LEAST ONE AGENT ON A STANDARDISED BIOFILM
PROCÉDÉ D'ESSAI ÉCOTOXICOLOGIQUE D'AU MOINS UNE SUBSTANCE ACTIVE SUR UN BIOFILM STANDARDISÉ

(43) Veröffentlichungstag der Anmeldung: 10.06.2020
(73) Patentinhaber: Justus-Liebig-Universität Gießen, 35390 Gießen (DE)
(72) Erfinder: Dr. Pohlon, Elisabeth, 35037 Marburg (DE); Drenker, Christian Oliver, 63225 Langen (DE)
(74) Vertreter: Stumpf, Peter

(56) Entgegenhaltungen:
- EHRMAN ET AL: "Fungi in a heavy metal precipitating stream in the Mansfeld mining district, Germany", SCIENCE OF THE TOTAL ENVIRONMENT, ELSEVIER, AMSTERDAM, NL, Bd. 389, Nr. 2-3, 24. Oktober 2007 (2007-10-24), Seiten 486-496, XP022349609, ISSN: 0048-9697, DOI: 10.1016/J.SCITOTENV.2007.09.004
- SOLE ET AL: "Aquatic hyphomycete communities as potential bioindicators for assessing anthropogenic stress", SCIENCE OF THE TOTAL ENVIRONMENT, ELSEVIER, AMSTERDAM, NL, Bd. 389, Nr. 2-3, 1. November 2007 (2007-11-01), Seiten 557-565, XP022349617, ISSN: 0048-9697, DOI: 10.1016/J.SCITOTENV.2007.09.010
- MANUELA ABELHO ET AL: "Effects of the fungicide pyrimethanil on biofilm and organic matter processing in outdoor lentic mesocosms", ECOTOXICOLOGY, Bd. 25, Nr. 1, 23. Oktober 2015 (2015-10-23), Seiten 121-131, XP055571381, GB ISSN: 0963-9292, DOI: 10.1007/s10646-015-1574-x
- FERNÁNDEZ DIEGO ET AL: "Does nutrient enrichment compensate fungicide effects on litter decomposition and decomposer communities in streams?", AQUATIC TOXICOLOGY, ELSEVIER, AMSTERDAM, NL, Bd. 174, 27. Februar 2016 (2016-02-27), Seiten 169-178, XP029475854, ISSN: 0166-445X, DOI: 10.1016/J.AQUATOX.2016.02.019
- CORCOLL NATÀLIA ET AL: "Effects of flow intermittency and pharmaceutical exposure on the structure and metabolism of stream biofilms", SCIENCE OF THE TOTAL ENVIRONMENT, ELSEVIER, AMSTERDAM, NL, Bd. 503, 10. Juli 2014 (2014-07-10), Seiten 159-170, XP029094575, ISSN: 0048-9697, DOI: 10.1016/J.SCITOTENV.2014.06.093
- ARTIGAS J ET AL: "Comparative sensitivity to the fungicide tebuconazole of biofilm and plankton microbial communities in freshwater ecosystems", SCIENCE OF THE TOTAL ENVIRONMENT, Bd. 468, 1. Januar 2014 (2014-01-01), Seiten 326-336, XP028795124, ISSN: 0048-9697, DOI: 10.1016/J.SCITOTENV.2013.08.074
- JAN DIJKSTERHUIS ET AL: "Effects of Seven Fungicides on Non-Target Aquatic Fungi", WATER, AIR, AND SOIL POLLUTION, KLUWER ACADEMIC PUBLISHERS, DO, vol. 222, no. 1 - 4, 28 May 2011 (2011-05-28), pages 421-425, XP019972971, ISSN: 1573-2932, DOI: 10.1007/S11270-011-0836-3

## Beschreibung

Die vorliegende Erfindung betrifft ein Verfahren zur ökotoxikologischen Testung von mindestens einem Wirkstoff an einem Biofilm aus aquatischen Pilzen. Dazu wird ein künstliches Substrat für aquatische Pilze zur standardisierten Besiedlung mit aquatischen Biofilmen bereitgestellt.

Mikrobielle Biofilme sind bekannt als Lebensgemeinschaften von Mikroorganismen, wie Bakterien, Pilzen und/oder Algen und Protozoen, welche sich an Oberflächen anheften und dort aufwachsen. Sie sind an die jeweilige Umgebung angepasst und verfügen über eine höhere Resistenz gegenüber Umgebungsbedingungen als unabhängige Einzelzellen bzw. Einzeller. Der Stoffwechsel von in Biofilmen organisierten Zellen unterscheidet sich von dem freier oder planktonischer Zellen. Biofilme sind charakterisiert dadurch, dass die Zellen von einer mikrobiell induzierten Matrix, meist aus Polysacchariden, umgeben sind, welche von einer wässrigen Lösung durchströmt wird.

Mikrobielle Biofilme können aus einzelnen Taxa oder aus Mischpopulationen verschiedener Mikroorganismenarten zusammengesetzt sein. Die in Biofilmen organisierten Mikroorganismen nutzen Stoffwechseleigenschaften der anderen Taxa oder deren Schutzmechanismen oder metabolisieren in Biofilmen Zellen oder Zellteile anderer Mikroorganismen. Dabei sind die Bildung und der Austausch sogenannter Signalmoleküle für die mikrobielle Kommunikation entscheidend. Die Biofilmbildung erfolgt in mehreren Stufen. Sie ist nicht nur von der Art der Mikroorganismen und deren Eigenschaften abhängig, sondern wird auch von den Oberflächeneigenschaften der zu besiedelnden Materialien sowie den Umgebungsbedingungen beeinflusst. Hierzu zählen neben dem Nährstoffangebot auch Wassergehalt, Temperatur oder die Strömungsbedingungen. Bei der Entwicklung eines Biofilms im aquatischen Ökosystem heften sich zunächst die frei schwimmenden planktonischen Zellen an eine Oberfläche an. Nach der Anheftung folgt die Bildung von Monolayern und Mikrokolonien. Unter Vermittlung von Adhäsinen kommt es zu einer ersten Bindung mit der Materialoberfläche. Die schleimartige Matrix besteht aus extrazellulären polymeren Substanzen, welche den Biofilm durchziehen und umgeben. Diese basiert überwiegend auf Polysacchariden, welche die Mikroorganismen selbst produzieren. Diese Struktur lässt sich auch durch kräftiges Spülen nicht mehr von der Oberfläche entfernen. Aus dem Biofilm werden einzelne Zellen oder Mikroorganismen freigesetzt, die sich wieder ansiedeln und einen neuen Biofilm bilden können. In Biofilmen können Mikroorganismen nicht nur Nährstoffe akkumulieren, sie bieten der Population auch Schutz vor chemischen und physikalischen Umwelteinflüssen wie beispielsweise Austrocknung, Prädation, schädliche Strahlung und Pestiziden. Auch in Gewässern erfüllen Biofilme wichtige Funktionen. Sie dienen dem Stoffumsatz und der Retention in den jeweiligen Gewässerabschnitten und bilden eine wichtige Nahrungsquelle für Protozoen und höhere Organismengruppen insbesondere für phytophage Weidegänger (Grazer) wie Nematoden, Egel, Schnecken, einige Insektenlarven und Fische.

Die Beschaffenheit des Biofilms, wie Dicke und Konsistenz kann mikroskopisch in *situ* oder durch Anfärben analysiert werden.

Funktionelle Aspekte, wie der Stoffumsatz werden bevorzugt durch EEA-Messungen (EEA=extrazelluläre Enzym-Aktivität) bestimmt. Diese Aktivitäten werden in Bezug zu den charakteristischen Mikroorganismengruppen gesetzt, da sie z.T. spezifisch sind, wie Peroxidasen und Phenoloxidase, welche nur von Pilzen gebildet werden. Die Biomasse von Pilzen wird durch die Messung der Ergosterol-Konzentration charakterisiert. Die Algenbiomasse wird mittels Chlorophyllmessungen ermittelt und die Gesamtbakterienzahl mikroskopisch nach Anfärben mit DNA-Farbstoffen wie beispielsweise SybrGreen. Diese Verfahren sind dem Fachmann bekannt.

Im Stand der Technik bekannte Testverfahren basieren darauf, dass ein meist flächig ausgebildeter Testkörper mit einem Biofilm besiedelt wird und dann der Besiedelungsgrad des Testkörpers unter verschiedenen Bedingungen oder unter Einfluss von Substanzen, welche den Biofilm potentiell beeinträchtigen können, ermittelt wird.

Dabei ist insbesondere die Bewertung von Wirkstoffen, die eine fungizide Wirkung haben, auf Biofilm bildende Organismen wie z.B. aquatische Pilze besonders schwer zu ermitteln, weil es keine standardisierten Biofilme und Testsysteme gibt. Dabei muss berücksichtigt werden, dass Fungizide nicht ausschließlich auf die Zielarten (Schadpilze) wirken, sondern auch auf Nichtzielarten, welche in einem Ökosystem positive Funktionen ausüben. Im Rahmen der Umweltverträglichkeitsprüfungen von Wirkstoffen wie Fungiziden während der Zulassungsverfahren, sollte somit auch deren Einfluss auf Nichtzielarten untersucht und berücksichtigt werden. Aquatische Pilze in Bächen, Flüssen, Seen oder Teichen haben besonders positive Eigenschaften im Hinblick auf das Selbstreinigungspotenzial von diesen Gewässern. Sie ernähren sich vom dort vorhandenen organischen Material. Terrestrisches Pflanzenmaterial (überwiegend Laub, Zweige, verholzte Streu) aus der Ufervegetation stellt eine wichtige Quelle für organisches Material in Flüssen und anderen verwandten Süßwasserökosystemen dar. Lignolytische (= verholzendes Material abbauende) aquatische Pilze gehören zu den ersten und am häufigsten vorkommenden biofilmbildenden Organismen, welche die verholzte Streu kurz nach dem Eintritt in die Flüsse besiedeln.

Die ersten Schritte der Zersetzung hängen vor allem von den Pilzen ab, da sie die einzigen Organismen sind, welche Lignin effektiv abbauen können. Zu diesem Zweck produzieren die aquatischen Pilze ligninmodifizierende extrazelluläre Enzyme wie Ligninperoxidase, Manganperoxidase oder Laccase (Phenoloxidase). Aquatische Pilze stellen dabei eine Verbindung zwischen verschiedenen trophischen Ebenen in aquatischen Nahrungsnetzen dar. Sie liefern Fragmente und Metabolite aus dem Abbau von Blattstreu als Nähr- und Energiequellen für andere Mikroorganismen und Primärproduzenten. Aquatische Pilze tragen auch zur Bildung von Huminstoffen bei, welche essentielle Langzeit-Kohlenstoffspeicher in Frischwasser-Ökosystemen darstellen. Hierbei kondensieren Metabolite des Ligninabbaus mit Metaboliten aus weiteren Abbauprozessen von organischen Substanzen. Zusätzlich machen Pilze die Blattstreu schmackhafter für Zerkleinerer (Schredder) wie Insektenlarven, Gammariden (z. B. *Gammarus fossarum*) oder Schnecken. Sie verändern die Struktur der Blattstreu und verbessern das Nährwertprofil mit mehr Proteinen und Lipiden durch die pilzliche Biomasse. Unverdauliche oder abschreckende Verbindungen von Blattstreu wie Tannine, ätherische Öle oder Lignin werden ebenfalls durch Pilze reduziert. Aufgrund des hohen Spektrums an (extrazellulären) Enzymen können aquatische Pilze auch organische Schadstoffe in Süßwassersystemen abbauen.

Wichtig sind hierfür die ligninmodifizierenden Enzyme (Peroxidasen, Laccase) und Cytochrom P-450 Monooxygenasen. Beispiele für Substanzen, die durch aquatische Pilze abgebaut werden können, sind polyzyklische aromatische Kohlenwasserstoffe wie Pyren und Insektizide wie Malathion oder Lindan. Einige aquatische Pilze sind auch in der Lage, Schwermetalle wie Cadmium zu binden.

Seit Ende der 1980er Jahre ist bekannt, dass der Einsatz von Fungiziden aquatische Pilze beeinträchtigen kann. Der größte Teil der Fungizidbelastung in Fließgewässern ist landwirtschaftlichen Ursprungs und gelangt nach der Anwendung auf angrenzenden Feldern als Abfluss oder Abdrift in die Fließgewässer. Die Konzentrationen von Fungiziden in Fließgewässern können Werte erreichen, die negative Auswirkungen auf aquatische Pilze und ihre Funktionen für Bachökosysteme haben.

Ökosystemrelevante Konzentrationen von Pentachlorphenol (PCP) können die Vielfalt der aquatischen Pilze durch Reduktion von Sporulation und Vermehrung verringern. Die Ökosystemfunktionen von aquatischen Pilzen, wie der Streuabbau und die Konditionierung von Blattstreu für Zerkleinerer (Laubschredder), werden auch durch ökosystemrelevante Konzentrationen von Chlorthalonil, Azoxystrobin, Tebuconazol und mehreren anderen Fungiziden beeinflusst. Obwohl bekannt ist, dass fungizide Pflanzenschutzmittel aquatische Pilze schädigen können, werden bei der Bewertung und Registrierung von Fungiziden in der Europäischen Union Nichtzielpilze nicht berücksichtigt. Das führt zu einer Unterschätzung der Auswirkung auf aquatische Pilze. Wirkstoffe für Pestizide müssen vom Panel on Plant Protection Products and their Residues (PPR) der Europäischen Behörde für Lebensmittelsicherheit (EFSA) für die legale Verwendung in der EU bewertet und registriert werden. Umweltverträglichkeitsprüfungen (ERA) zur Auswirkungen von Pestiziden auf Wasserorganismen müssen auf der Grundlage der "Guidance on tiered risk assessment for plant protection products for aquatic organisms in edge-of-field surface waters" der EFSA-PPR (2013) erfolgen. Die Umweltrisikobewertung folgt einem abgestuften Ansatz, der von Einzelspezies-Tests bis hin zu komplexen Mesokosmen- oder Feldstudien für akute und chronische Wirkungen reicht. In dem Leitfaden werden auch die Referenzarten für Tests an Wasserorganismen der folgenden Taxa aufgeführt: Algen, Gefäßpflanzen, Wirbellose und Wirbeltiere. Für aquatische Mikroorganismen, einschließlich Pilzen, gibt es weder Referenzarten noch empfohlene Testverfahren, die zur Risikobewertung verwendet werden. Die regulatorisch zulässigen Konzentrationen (RAC) von Fungiziden in Bezug auf aquatische Pilze werden auf der Grundlage der empfindlichsten genannten Testspezies berechnet. Dieser Ansatz ist problematisch, da festgestellt wurde, dass Fungizidkonzentrationen unterhalb des RAC (insbesondere für höherrangige ERA) für einige nach den aktuellen ERA-Verfahren berechneten Fungizid-Konzentrationen für aquatische Pilze schädlich sind. Darüber hinaus beeinflusst eine Beeinträchtigung von aquatischen Pilzen auch große Teile des Nahrungsnetzes und des Energiekreislaufs in Süßwassersystemen.

Der Stand der Technik kennt aus Ehrman et al.: "Fungi in a heavy metal precipitating stream in the Mansfeld mining district, Germany", Science of the total environment, Elsevier, Amsterdam, NL, Bd. 389, Nr. 2-3, 24.10.2007 (2007-10-24) (D1) ein Verfahren zur ökotoxikologischen Bewertung verschmutzter Gewässer. Hierzu wurde Erlenlaub gesammelt, in Stücke von 1,5 cm Durchmesser geschnitten, getrocknet und in durchlässige Nylonbeutel verpackt. Diese Beutel wurden dann in die Gewässer gehängt und zu verschiedenen Zeiten über einen Verlauf von 28 Tagen wieder herausgenommen und untersucht. Es wurde gezeigt, dass eine Besiedelung und ein Abbau der Blätter durch Pilze in Abhängigkeit stand zum Verschmutzungsgrad der Gewässer mit Schwermetallen. Das Pilzwachstum wurde durch Messung des Ergosterolgehaltes bestimmt. In niedrig verschmutzten Gewässern kam es zu einer schnellen Bildung eines Biofilms aus Pilzen, Bakterien und anderen Mikroorganismen, was zu einem schnellen Abbau der Blätter führte. Bei hoher Verschmutzung mit Schwermetallen waren Besiedlung und Abbau deutlich verlangsamt.

Der Stand der Technik kennt aus Abelho et al.: "Effects of the fungicide pyrimethanil on biofilm and organic matter processing in outdoor lentic mesocosms", Ecotoxicology, Bd. 25, Nr.1,23 Oktober 2015 (2015-10-23), Seiten 121-131 (D3) die Untersuchung des Effekts des Fungizids Pyrimethanil auf die Ausbildung von Biofilmen und die Zersetzung von Erlenlaub in wässrigen Ökosystemen. Hierzu wurde Erlenlaub gesammelt, getrocknet und in durchlässige Netzbeutel verpackt. Diese wurden am Boden von 1,5 m³ großen Behältern angeordnet, welche Sediment, Wasser und aquatische Lebewesen/Pflanzen enthielten. Pyrimethanil wurde dem System zugesetzt und das Erlenlaub nach 42 Tagen Inkubation untersucht. Das Pilzwachstum wurde durch Messung des Ergosterolgehaltes bestimmt. Der Einfluss von Pyrimethanil auf den Zersetzungsgrad der Blätter wurde nach 274 Tagen bestimmt. Die Entwicklung eines Biofilms wurde mit Hilfe von Plastikplatten untersucht.

Der Stand der Technik kennt aus Diego et al.: "Does nutrient enrichtment compensate fungicide effects on litter decomposition and decomposer communities in streams?", Aquatictoxicology, Elsevier, Amsterdam, NL, Bd.174, 27.02.2016 (2016-.02-27), Seiten 169-178 (D4) die Untersuchung des Einflusses von verschiedenen Fungiziden auf die Zersetzung von Biomasse in aquatischen Systemen. Hierzu wurde Erlenlaub gesammelt und in durchlässige Beutel verpackt. Diese wurden zunächst in Fliessgewässer eingebracht, um eine mikrobielle Kolonisierung der Blätter zu erzielen. Die Beutel wurden dann in künstliche Fliesskanäle eingebracht und dort mit verschiedenen Fungiziden in Kontakt gebracht. Es konnten im Versuchszeitraum keine signifikanten Effekte auf die Zersetzung des organischen Materials festgestellt werden.

Der Stand der Technik kennt aus Artigas et al.: "Comparative sensitivity to the fungicide tebuconazole of biofilm and plankton microbial communities in freshwater ecosystems", Science of the total environment, Bd. 468, 01.01.2014 (2014-01-01), Seiten 326-336 (D6) die Untersuchung des Effekts von Tebuconazol auf Biofilme und planktonische Organsimen in wässrigen Ökosystemen. Die Biofilme wurden aus Flüssen und Seen durch Kolonisierung von Glaskacheln erhalten. Während die Zahl der Bakterien, Algen, Pilze und Protozoen in Biofilmen durch Einwirkung von Tebuconazol abnahm, wurde zumindest für planktonische Bakterien eine Zunahme verzeichnet. Planktonische Pilze und Protozoen unterlagen ebenfalls einer Abnahme.

Der Stand der Technik kennt aus Dijksterhuis et.al.: "Effects of seven fungicides on non-target aquatic fungi", Water, air, and soil pollution, Kluwer Academiuc Publishers, Do, Bd. 222, Nr. 1-4, 28.05.2011 (2011-05-28). Seiten 421-425 (D7) ein Verfahren zur ökotoxikologischen Bewertung von Fungiziden hinsichtlich ihrer Auswirkungen auf aquatische Pilze. Hierzu werden die Pilze zunächst auf Malzextrakt-Agar kultiviert. Nach 12 Tagen werden die Konidien geerntet und dann in verschiedenen Medien inkubiert. Nach 24-stündiger Inkubation werden die Fungizide zugesetzt und der Effekt auf die Pilzzellen evaluiert.

Allerdings handelt es sich bei den bekannten Verfahren nicht um standardisierbare Verfahren zur ökotoxikologischen Testung von mindestens einem Wirkstoff an einem Biofilm aus aquatischen Pilzen.

### Aufgabe

Aufgabe der Erfindung ist es, die Probleme im Stand der Technik zu beheben und ein besser standardisierbares Verfahren zur ökotoxikologischen Testung von mindestens einem Wirkstoff an einem Biofilm aus aquatischen Pilzen bereitzustellen.

### Lösung

Die Aufgabe wird durch ein Verfahren gemäß Anspruch 1 gelöst. Vorteilhafte Weiterbildungen der Erfindung sind in den Unteransprüchen beschrieben.

Das Verfahren zur ökotoxikologischen Bewertung von mindestens einem Wirkstoff hinsichtlich der Auswirkungen auf einen Biofilm aus aquatischen Pilzen umfasst die folgenden Schritte:
i) Bereitstellung eines Trägers mit künstlichem Substrat, wobei das Substrat Erlenlaub-Agar umfassend 4% (Gew./Vol.) getrocknete, zerkleinerte Erlenblätter und 4% Agarose umfasst.
ii) In Kontaktbringung des Trägers mit dem künstlichen Substrat mit Wasser, welches ein Sporeninokulum aquatischer Pilze aufweist, so dass sich ein Biofilm aus aquatischen Pilzen auf dem Träger mit dem künstlichen Substrat bilden kann.
iii) Zugabe des mindestens einen zu testenden Wirkstoffs in das Wasser gemäß Schritt ii).
iv) Bestimmung der Biomasse aus aquatischen Pilzen im Biofilm aus Schritt ii). v) Bestimmung des Stoffumsatzes der aquatischen Pilze im Biofilm aus Schritt ii) wobei der Einfluss mindestens eines Wirkstoffs auf die Werte aus Schritt iv) und/oder aus Schritt v) des Biofilms im Vergleich zu einer Vergleichsprobe erfasst werden, wobei die Vergleichsprobe anstatt des Wirkstoffes die gleiche Menge z.B. filtriertes und autoklaviertes Wasser oder bekannte Referenzsubstanzen aufweist.

Erlenlaub-Agar kann beispielsweise 1-10% (Gew./Vol.) getrocknete, zerkleinerte Erlenblätter und 1-10% Agarose umfassen. Anspruchsgemäss umfasst der verwendete Erlenlaub-Agar 4% (Gew./Vol.) getrocknete, zerkleinerte Erlenblätter und 4% Agarose.

Der Träger ist ein Objektträger aus Glas. Es wird bevorzugt, dass der Objektträger wenigstens an der dem künstlichem Substrat zugewandten Seite aufgeraut oder angeschliffen ist. Die Kontaktbringung des Trägers mit dem künstlichen Substrat erfolgt mit Wasser welches ein natürliches Sporeninokulum aquatischer Pilze aufweist, so dass sich ein Biofilm aus aquatischen Pilzen auf dem Träger mit dem künstlichen Substrat bilden kann. Zum Beispiel stammt das Wasser aus einem Referenzgewässer. Alternativ wird Wasser eingesetzt, welches ein gezieltes Sporeninokulum mit einer oder mehrerer Pilzspezies umfasst.

Die Kontaktbringung des Trägers mit dem künstlichen Substrat mit Wasser erfolgt innerhalb von Fließrinnen oder Mesokosmen oder in Gewässern welche in Klimakammern mit standardisierbaren Bedingungen oder in halboffenen Gebäuden mit seminatürliche Bedingungen oder im Freiland platziert sind.

Die Kontaktbringung des Trägers mit dem künstlichen Substrat und Wasser (inklusive Sporeninokulum) gemäß Schritt ii) erfolgt innerhalb eines Inkubationszeitraumes der künstlichen Substrate von 9-14 Tagen.

Die Bestimmung der Menge der Biomasse der aquatischen Pilze des Biofilms in Schritt iv) erfolgt durch die Messung der Ergosterol-Konzentration.

Die Bestimmung des Stoffumsatzes im Biofilm in Schritt v) erfolgt durch Messung der extrazellulären Enzymaktivitäten von Phenoloxidase und Peroxidasen (pilzspezifische Enzymen), sowie α-Glukosidase, β-Glukosidase, Exocellulase, β-Xylosidase, Phosphatase und/oder Aminopeptidase.

Der mindestens eine Wirkstoff ist ein natürlicher oder chemischer Stoff, insbesondere ausgewählt aus der Gruppe Pestizide, Fungizide, Herbizide, Insektizide, Medikamente, Antibiotika, Dünger, Schwermetalle, Salze, endokrine Disruptoren (hormonaktive Substanzen) und/oder Mikroplastik.

Das Verfahren kann verwendet werden zur Ermittlung von durch mindestens einen Wirkstoff hervorgerufene akute (kurzfristige, kurzzeitige) oder chronische (langfristige langzeitige) Wirkung auf aquatische Pilze in einem Gewässer.

Diese Erfindung bietet erstmals die Möglichkeit zur ökotoxikologischen Testung von mindestens einem Wirkstoff wie z.B. einem Fungizid oder einem Gemisch aus mehreren Wirkstoffen, hinsichtlich der Wirkung auf aquatische Pilze unter standardisierten und replizierbaren Bedingungen. Das Verfahren kann unter Laborbedingungen, aber auch im Freiland angewendet werden.

Diese Erfindung bietet des Weiteren die Möglichkeit zur Anpassung an die Testung von Wirkstoffen auf Bakterien und Algen und kann daher später als multiples Testverfahren verwendet werden.

Im Vordergrund steht bei diesem Verfahren die ökotoxikologische Bewertung von Auswirkungen anthropogen ausgebrachter Wirkstoffe insbesondere Fungizide auf die Struktur und Funktion aquatischer Pilze. Insbesondere wird ein Verfahren zur ökotoxikologischen Bewertung von Fungiziden hinsichtlich ihrer Auswirkungen auf aquatische Pilze bereitgestellt.

Fungizide gehören in die größere Gruppe der als Pestizide bekannten Wirkstoffe und Substanzen, die nach heutigem Verständnis unter Anderem als Pflanzenschutzmittel zur Schädlingsbekämpfung eingesetzt werden. Typische Vertreter der Fungizide sind heute beispielsweise Azole (wie z.B. Benzimidazole, Triazole, und Imidazole) oder Morpholine (wie z.B. SBI-Fungizide), Strobilurine (wie z.B. Picoxstrobin, Azoxystrobin), Chinoline, Anilino-Pyrimidine, Oxazolidindione oder Carbonsäureamide.

Voraussetzung der Erfindung war es, ein standardisiertes künstliches Substrat zur Besiedlung durch eine definierte Biofilmgemeinschaft für ökotoxikologische Tests an aquatischen Pilzen unter Labor- und Freilandbedingungen zu entwickeln.

Dies erfolgte unter Verwendung von Objektträgern, wie sie für die Mikroskopie bekannt sind, da diese bereits erfolgreich für Untersuchungen an epilithischen Prokaryonten und Algen in aquatischen Biofilmen eingesetzt werden. Künstliche Oberflächen, wie Objektträger oder Tonfliesen bieten eine definierte Besiedlungsfläche und Oberflächeneigenschaften für die Biofilmkolonisation im Gegensatz zu natürlichen Substraten wie Holz oder Steinen.

Künstliche Substrate stellen einen geeigneten Ansatz für standardisierte ökotoxikologische Tests an aquatischen Pilzen dar. Studien über die ökotoxikologische Wirkung von Fungiziden auf aquatische Pilze sind selten und basieren bisher in der Regel auf der Besiedlung von Blattstreu.

Nahezu alle Studien zur Ökologie der aquatischen Pilze basieren ebenfalls auf der Verwendung von Blattstreu. Die hohe Variabilität der Blätter auch von derselben Art oder Pflanze ist der Reproduzierbarkeit abträglich.

Es ist bekannt, dass die intraspezifischen Variationen der Blattzusammensetzung im gleichen Bereich liegen wie die interspezifischen Variationen. Für Untersuchungen, welche Blattstreu als Substrat geeignet ist, ist oft die Bestimmung des Blatttrockengewichts als Referenz für die gemessenen Parameter erforderlich. Dies verursacht zusätzliche zeitaufwändige Schritte bei der Probenverarbeitung. Aquatische Pilze gehören zu den ersten Besiedlern von verholzter terrestrischer Streu in Bächen, aber die Pilzkolonisation dauert auf natürlichem Substrat mit etwa 30 Tage sehr lange, bis sie auf dem Blattstreu ihren Höhepunkt erreicht hat und noch länger auf holzigem Streu. Dies ist sehr nachteilig für die Beurteilung der Auswirkungen von Pestiziden auf aquatische Pilze bei der Bewertung und Registrierung von Pestiziden in einem akzeptablen Zeitrahmen.

Es wird erfindungsgemäß ein künstliches Substrat bereitgestellt, dass die folgenden Eigenschaften aufweist:
a) Eignung für die Anwendung in Fließrinnen oder Mesokosmen oder Fließgewässern für eine Besiedlung mit aquatischen Pilzen für mindestens einen Monat, ohne dass sich das Substrat ablöst
b) geringe Unterschiede zwischen den Proben durch homogene Substratzusammensetzung
c) eine definierte Oberfläche (7,6 x 2,6 cm ~ 19,76 cm²) als geeignete Referenz anstelle von Blatttrockengewicht
d) schnelles Erreichen einer maximalen Besiedlung durch aquatische Pilze, da Laub ihr natürliches Substrat ist

In eigenen wissenschaftlichen Experimenten wurde die Besiedelung mit aquatischen Pilzen auf verschiedenen organischen Beschichtungen für Objektträger über Zeiträume von 1-20 Tagen getestet, sowie die Haftungseigenschaften der Beschichtungen über einen Monat. Diese Substrate bieten eine schnelle Kolonisation mit aquatischen Pilzen (sie bilden Mykobiofilme) von weniger als 14 Tagen. Dazu wurden Träger mit künstlichem Substrat beschichtet, so dass eine Beschichtung vorliegt.

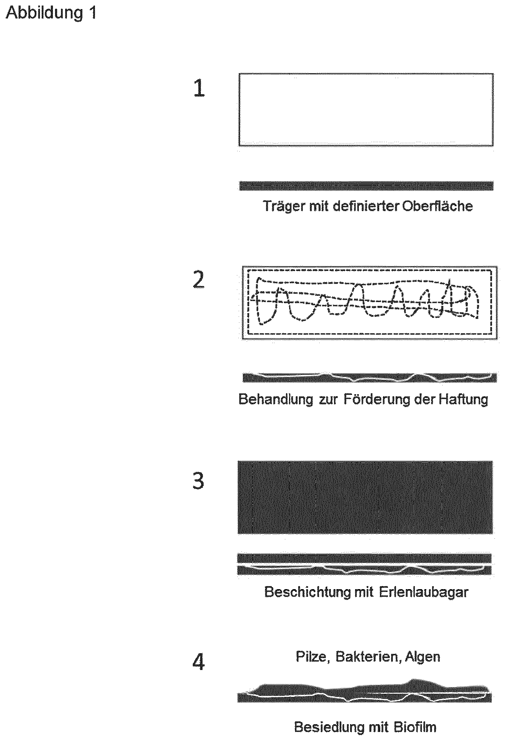

Als Träger wurden Objektträger aus Glas verwendet. Die Objektträger sind an der Oberfläche aufgeraut oder angeschliffen, so dass das künstliche Substrat dort besser haftet. Das künstliche Substrat haftet nachweislich für mindestens einen Monat in laminarer und nichtlaminarer Wasserbewegung. Die Oberfläche der Objektträger wird an der dem künstlichen Substrat zugewandten Seite z.B. mit einer Schleifvorrichtung z.B. einem Handschleifer (z.B. Dremel) mit einer geeigneten diamantbeschichteten Trenn- / Schleifscheibe aufgeraut. Als Schleifbild werden ein schmaler Schliff um den Rand und ein partieller Freiformschliff erstellt (Abbildung 1). Dieses Muster wird für die beste Kombination aus langlebigstem Beschichtungsverbund und geringstem Schleifaufwand gewählt. Vor der Beschichtung werden alle Objektträger für mind. vier Stunden bei 550 °C behandelt (z.B. in einem Muffelofen), um mögliche organische Rückstände zu verbrennen.

Als Substrat wird erfindungsgemäß Erlenlaub-Agar zur Beschichtung der Objektträger verwendet, welcher sich gegenüber einem supplementierten Malz-Agar-Substrat und unbeschichteten Objektträgern als optimal herausgestellt hat.

Durch das Hinzufügen von einer definierten Menge zerkleinerten Erlenblättern kann die Unzuverlässigkeit der herkömmlichen Verfahren unter Verwendung von ganzen Blättern umgangen werden, ohne dass man auf Laub als natürliches Substrat für Pilze verzichten muss. Während eines Zeitraumes von 20 Tagen Biofilmbildung in Fließrinnen waren alle Beschichtungen stabil, es kam zu keiner Ablösung vom Objektträger.

### Ausführungsbeispiele

**Tabelle 1: Herstellung des künstlichen Substrates aus Erlenlaub-Agar mit den bevorzugten Konzentrationen.**

| **Stoff** | **Menge** |
|---|---|
| getrocknete Erlenblätter (zerkleinert) | 4 % (Gew./Vol.) |
| Agarose | 4 % (Gew./Vol.) |
| deionisiertes Wasser | Ad 100% (Vol.) |

**Tabelle 2: Herstellung des supplementierten Malz Agar als Vergleichsprobe.**

| **Stoff** | **Menge** |
|---|---|
| Malzextrakt | 1 % (Gew./Vol.) |
| Hefeextrakt | 1 % (Gew./Vol.) |
| Pektin | 0,5 % (Gew./Vol.) |
| Glucose | 1% (Gew./Vol.) |
| Agarose | 5 % (Gew./Vol.) |
| deionisiertes Wasser | Ad 100% Vol. |

Alle Inhaltsstoffe werden mit einem Mixer gemischt, homogenisiert und die resultierende Suspension sterilisiert. Das Substrat wird als Beschichtung auf die Objektträger aufgetragen (z.B. mittels Pinsel). Für die Beschichtung wird pro Objektträger jeweils mind. 1,0 mL Erlenlaub-Agar verwendet.

Abbildung 1 zeigt ein Schema zur Beschichtung des Objektträgers mit Erlenlaub-Agar und die Besiedlung von aquatischen Pilzen als Biofilm.

Die Besiedlung und Funktion der Biofilme wird durch Bestimmung der Ergosterol-Konzentrationen und der extrazellulären Enzymaktivitäten (EEA) analysiert.

Der Einfluss auf die Funktion von aquatischen Pilzen durch den mindestens einen Wirkstoff z.B. ein Fungizid oder das Wirkstoffgemisch lässt sich in Tests über eine Veränderung der EEA sowie der Ergosterolkonzentration ermitteln.

Die mit künstlichem Substrat beschichteten Objektträger wurden in Fließrinnen mit gleichbleibenden Lichtverhältnissen getestet. Die für den Test verwendeten Fließrinnen, mit Innenmaßen von 158 x 10 x 15 cm (Länge x Breite x Höhe) und einer Kapazität für 106 Objektträger.

Sie waren mit einem eigenen 31-Liter-Wassertank verbunden. Die Wasserkreislaufführung erfolgte über Tauchpumpen (S1000; Resun). Die Strömungsgeschwindigkeit in den Fließrinnen betrug 0,014 ± 0,0032 m s⁻¹ (Flo - Mate 2000; Hach - Marsh McBirney). Die Fließrinnen wurden mit Wasser aus einem Mittelgebirgsbach südwestlich von Gießen befüllt, um ein natürliches Sporeninokulum aquatischer Pilze zu erhalten. Die Fließrinnen wurden perfundiert (mit Wasser durchströmt). Einmal pro Woche wurde etwa die Hälfte der Wassermenge durch Frischwasser ersetzt. In einem Beschichtungsauswahl-Experiment wurden die Objektträger mit dem Erlenlaub-Agar auf ihre Fähigkeit untersucht, die Besiedelung mit Biofilmen, im Vergleich zu unbeschichteten Objektträgern zu beschleunigen. Als Vergleichsprobe wird ein Objektträger beschichtet mit supplementiertem Malz-Agar eingesetzt. Nach 20 Tagen Kolonisation wurden Proben für Analysen entnommen. Von jeder Beschichtung bzw. aus jedem Strömungskanal wurden fünf Objektträger für die Bestimmung der Enzymaktivität und fünf Objektträger für die Ergosterol-Messung entnommen. Biofilme wurden durch EEA-Messungen und die Messung der Ergosterol-Konzentration charakterisiert. Dabei zeigte sich, dass die Beschichtung mit Erlenlaub-Agar optimale Bedingungen für die Besiedlung durch aquatische Pilze darstellt. Ein Kolonisationsdynamik-Experiment mit Erlenlaub-Agar als Substrat auf Objektträgern zeigte, dass zur Untersuchung von aquatischen Pilzen bei Probennahmen am Tag 1, 3, 5, 7, 9, 11 und 14 drei Objektträger für den EEA und drei Objektträger für die Ergosterol-Messung ausreichen und dass nach 9 Tagen eine zuverlässige Pilzbiomasse nachgewiesen werden konnte. Das heißt, 9 Tage Besiedlung reichen für eine wirksame Testung aus. Für Untersuchungen zu den Auswirkungen mindestens eines Wirkstoffes oder eines Wirkstoffgemisches werden die mit Erlenlaub-Agar beschichteten Objektträger zunächst über 9-14 Tagen zur Besiedelung mit Biofilmen in den Fließrinnen (oder Mesokosmen oder Fließgewässern) inkubiert, bevor eine Applikation des mindestens eines Wirkstoffes oder Wirkstoffgemisches z.B. eines Fungizides erfolgt. Die Messung erfolgt gegen Biofilmproben aus Vergleichsproben. Vergleichsproben sind Kontrollansätze, welchen anstatt des Wirkstoffes die gleiche Menge z.B. filtriertes und autoklaviertes Wasser aufweisen oder bekannte Referenzsubstanzen.

### Probenvorbereitung für EEA-Messung

Aus jeder Fließrinne werden drei Objektträger entnommen. Die Biofilme werden mit gefiltertem (0,2 µm Celluloseacetatfilter) und autoklaviertem (121 °C, 30 Minuten) Wasser mit einem Skalpell von den Objektträgern gelöst und in kleine Stücke zerteilt.

Diese Stücke werden in ein Homogenisierungsbecherglas überführt. Jeder Objektträger wird mit 5 mL Wasser gespült und die Wassermenge wird im gleichen Becherglas gesammelt. Dann wird Wasser hinzugefügt, bis ein Endvolumen von 70 mL erreicht ist. Die Biofilmsuspension wird für 1 Minute mit einem Dispergierer homogenisiert. Durch die separate Probenahme und Probenaufbereitung nach Fließrinne gewinnt man 4 Replikate, um eine spätere statistische Auswertbarkeit zu gewährleisten.

### Messung der potentiellen EEA

Die potentielle EEA der pilzspezifischen Enzyme Phenoloxidase und Peroxidase wurden photometrisch mittels einem L-3,4-Dihydroxyphenylalanin (L-DOPA)-Oxida-tionsassay nach der dem Fachmann bekannten Methode von Hendel, Sinsabaugh & Marxsen aus 2005 bestimmt.

Die Aktivitäten von α-Glukosidase, β-Glukosidase, Exocellulase, β-Xylosidase, Phosphatase und Aminopeptidase werden fluorimetrisch unter der Verwendung fluorigener Enzymsubtrate mittels der Methode von Hendel & Marxsen aus 2005 gemessen. Folgende Abweichungen gegenüber der Methode von Hendel & Marxsen aus 2005 wurden verwendet: Die Konzentration der Substrat-Stammlösungen betrug 2 µmol mL⁻¹. Der Glycinpuffer setzte sich wie folgt zusammen: 50 mmol L⁻¹ Glycinpuffer (6,43 g NaOH und 2,95 g Glycin in 1 L deionisiertem und autoklaviertem Wasser, pH 10,4).

**Tabelle 1: Extrazelluläre Enzyme mit entsprechenden Modell-Substraten zur Messung der extrazellulären Enzymaktivität sowie deren natürliche Substrate.**

| **Enzym** | **Substrat für EEA** | **Natürliches Substrat** |
|---|---|---|
| α-Glucosidase | MUF-α-D-Glucosid | Maltose, Stärke |
| β-Glucosidase | MUF-β-D-Glucosid | Cellobiose |
| Exocellulase | MUF-β-D-Cellubiosid | Cellulose (setzt Cellubiosee frei) |
| β-Xylosidase | MUF-β-D-Xylosid | Hemicellulose, Xylane |
| Phosphatase | MUF-Phosphat | Polyphosphatase |
| Aminopeptidase | L-Leucin-AMC | Peptide, Proteine |
| Phenoloxidase | L-DOPA | Lignin |
| Peroxidasen | L-DOPA + H₂O₂ | Lignin |

### Isolierung des Biofilms für die Ergosterolextraktion

Biofilme von 3 Objektträgern wurden mittels Skalpell von diesen gelöst und die Objektträger mit 1 mL deionisiertem Wasser nachgespült. Die abgelösten Biofilme werden inklusive des Wassers zum nachspülen der Objektträger in 50 mL Polypropylenröhrchen mit Schraubverschluss übertragen.

Nach Zugabe von Wasser zu einem Endvolumen von 10 mL werden die Biofilme ca. eine Minute lang mit einem Dispergierer homogenisiert, bei -80 °C eingefroren und 3 Tage gefriergetrocknet.

### Biomassebestimmung der aquatischen Pilze

Es ist allgemein bekannt, dass die Ergosterol-Konzentration ein Maß für die Pilzbiomasse ist. Ergosterol ist ein Zellwandbestandteil. Die Messung ist dem Fachmann bekannt. Der Einfluss auf die Abundanz / Biomasse von aquatischen Pilzen durch den mindestens einen Wirkstoff z.B. ein Fungizid oder durch das Wirkstoffgemisch lässt sich über eine Veränderung der Ergosterolkonzentration feststellen.

### Ergosterol-Extraktion

Ergosterol wird mit einem mikrowellenunterstützten Verfahren aus den Biofilmproben extrahiert. Gefriergetrocknete Biofilmproben werden in 30 mL Schraubverschlussgläser mit PTFE-Dichtungen überführt und 4 mL Methanol und 1 mL wässrige NaOH-Lösung (2 mol L⁻¹) hinzugefügt.

Die Proben werden zweimal für 8 Sekunden bei 1250 W (2450 MHz) in der Mikrowelle erhitzt und nach dem ersten Mikrowellenzyklus für 3 Minuten und 15 Minuten nach dem zweiten bei Raumtemperatur abgekühlt. Dann werden 2 mL HCl (1 mol L⁻¹) und 2 mL Methanol hinzugefügt. Die Extraktion erfolgt durch dreimaliges Waschen mit 2 mL Pentan und Überführen der Pentanphasen in Glasreagenzgläser. Um eine bessere Phasentrennung und weniger Feststoffe im Extrakt zu erreichen, wurden die Proben 10 Minuten lang bei 2500 U/min (1057 G) zentrifugiert, bevor der Pentanüberstand zum dritten Mal abgenommen wird. Pentan wird durch Erhitzen der Reagenzgläser in einem Wasserbad auf 55 °C verdampft. Die Rückstände werden durch zweimaliges Waschen mit 0,5 mL Methanol in 1,5 mL HPLC-Vials übertragen. Um die Extraktionseffizienz abzuschätzen, wird mit jeder Mikrowellencharge zusätzlich eine Ergosterol-Kontrolle extrahiert. Die Ergosterol-Kontrolle enthielt 50 µL Ergosterol-Stammlösung (11 mg Ergosterol in 50 mL Methanol) anstelle einer Biofilmprobe. Die Extrakte werden bis zur HPLC-Analyse bei 2 °C gelagert.

### Ergosterol-Messungen

Die Messung der Ergosterol-Konzentrationen des Biofilms erfolgte nach der dem Fachmann bekannten Methode von Gessner aus 2005.

### Ergebnisse aus Testreihen zur Evaluierung des Substrates und der Besiedlungszeiträume

Eigene Versuche haben gezeigt, dass der Erlenlaub-Agar gegenüber der Vergleichsprobe (supplementierter Malzagar) und unbeschichteten Objektträgern in Hinblick auf die Kolonisation mit aquatischen Biofilmen überlegen ist (Abbildungen 2+3). Während einer 14-tägigen Kolonisation von mit Erlenlaub-Agar-beschichteten Objektträgern mit aquatischen Biofilmen zeigte sich eine graduelle Steigerung sowohl der Enzymaktivitäten, als auch der Ergosterolkonzentration. Ein deutlicher Anstieg zeigte sich innerhalb der zweiten Woche (Abbildungen 4+5).

Eine Beschichtung für Objektträger wird bereitgestellt, welche sich durch ihre Befestigungseigenschaften, ihre Stabilität gegen Ablösung in Gewässern und ihre Eignung für die Besiedlung durch Pilze auszeichnet. Die mit Erlenlaub-Agar beschichteten Träger wurden unter semi-natürlichen Bedingungen in Fließgerinnen für die Bildung des Biofilms aus aquatischen Pilzen und deren EEA und Ergosterolkonzentration als Maße für die Ökosystemfunktion getestet. Die signifikant höheren Ergosterol-Konzentrationen in Biofilmen aus Erlenlaub-Agar-Beschichtungen zeigten eine höhere Besiedlung mit aquatischen Pilzen im Vergleich zu supplementiertem Malz-Agar-Beschichtungen oder unbeschichteten Objektträgern. Die Analyse der potenziellen EEA zeigten auch, dass Biofilme von Erlenlaub-Agar-Beschichtungen signifikant höhere Aktivitäten der pilzspezifischen ligninabbauenden Phenoloxidase und der kohlenstoffabbauenden Enzyme aufweisen. Die hohen Aktivitäten der unspezifischen Enzymaktivitäten implizieren, dass auch andere Organismengruppen als Pilze, wie beispielsweise Bakterien von dem Erlenlaub-Agar profitieren. Beschichtungen aus in der Pilzzucht häufig verwendeten Nährstoffen wie Malzextrakt, wie sie im supplementierten Malzagar verwendet wurden, sind für die Verwendung in aquatischen Systemen ungeeignet.

Malzextrakt besteht zu 92 % (Trockengewicht) aus Kohlenhydraten, meist Mono- und Disacchariden, die hochgradig wasserlöslich sind oder von Bakterien und Pilzen in kürzester Zeit aufgenommen werden. Die Erlenlaub-Agar-Beschichtung enthält als Nährstoffkomponente nur Erlenblätter. Getrocknete Erlenblätter enthalten an für aquatische Pilze relevante Nährstoffe durchschnittlich ca. 15 % Cellulose, 14 % Lignin, 14 % Proteine und 0,16 % Gesamtphosphor. Diese Komponenten sind schwer wasserlöslich. So liefert die Erlenlaub-Agar-Beschichtung reichlich und über einen längeren Zeitraum Nährstoffe für Organismen (u.a. aquatische Pilze) mit cellulytischer und lignolytischer Enzymsegregation.

Vergleiche mit anderen Studien, welche aquatische Biofilme aus Holz und Blattstreugut untersuchten, zeigen, dass vergleichbare oder höhere Enzymaktivitäten und Ergosterol-Konzentrationen auf Erlenlaub-Agar in deutlich kürzerer Zeit erzielt werden können.

Das erfindungsgemäße Verfahren weist viele Vorteile auf. So kann z.B. der Zeitraum für die Bildung eines Biofilms auf unter 14 Tage reduziert werden, was ein ausreichender Zeitrahmen für Tests ist. Die Ergebnisse der Aktivitäten für die pilzspezifischen Enzyme Phenoloxidase und Peroxidase, die in früheren Studien nach drei bis vier Wochen an Blattstreu gemessen wurden, wurden innerhalb von 9 bis 14 Tagen erreicht. Weiterhin ließen sich die Variabilität der Substratzusammensetzung reduzieren und die Homogenität erhöhen. Zusammenfassend lässt sich sagen, dass das entwickelte Substrat alle notwendigen Eigenschaften für erfolgreiche ökotoxikologische Studien an aquatischen Pilzen bietet. Sie bilden eine fundierte Grundlage für die Entwicklung der dringend benötigten standardisierten Tests zur Risikobewertung der Auswirkungen von Fungiziden auf aquatischen Pilzen. Neben der Verwendung für ökotoxikologische Anwendungen kann dieses Substrat auch für alle Arten von ökologischen Untersuchungen an aquatischen Biofilmen von Nutzen sein.

Erstmals ist die Ermittlung einer durch mindestens einen Wirkstoff hervorgerufene Wirkung auf aquatische Pilze in einem Gewässer unter standardisierten bzw. replizierbaren Bedingungen möglich.

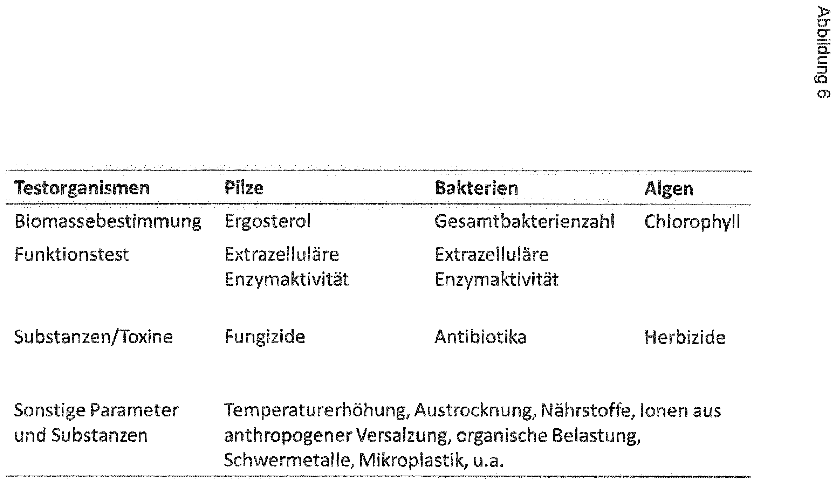

Das erfindungsgemäße ökotoxikologische Testverfahren zur Bewertung des mindestens einen Wirkstoffes auf Biofilme aus aquatischen Pilzen kann auch auf die Bewertung von Biofilmen anderer Organismen wie Bakterien und Algen übertragen werden (Abbildung 6).

### Abbildungslegenden und Bezugszeichenliste

**Abb. 1** zeigt im Vergleich einen geschliffenen Objektträger, sowie beschichtete Objektträger mit Erlenlaub-Agar.

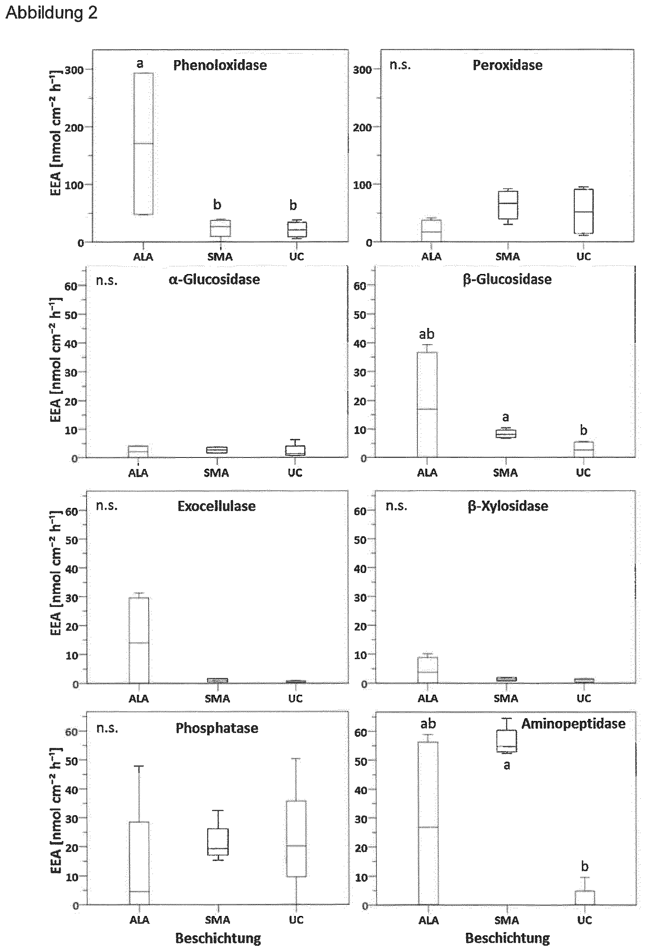

**Abb. 2** zeigt die extrazellulären Enzymaktivitäten nach 20 Tagen Kolonisation von künstlichen Substraten auf Basis von Erlenlaub-Agar (ALA; n=4) und zum Vergleich mit supplementiertem Malz-Agar (SMA; n=4) bzw. auf unbeschichteten Objektträgern (UC; n=4). Kleine Buchstaben über den Kästchen zeigen signifikante Unterschiede an (Mann-Whitney U-Test, α: 0,05), während n.s. keine signifikanten Unterschiede anzeigt. Boxplots: Box = 25 % - 75 % Quantil, Whisker = höchste und niedrigste Werte, horizontaler Balken = Median.

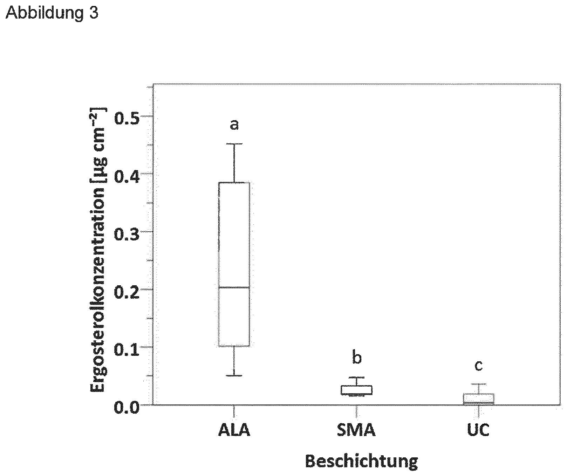

**Abb. 3** zeigt die Ergosterol-Konzentrationen in den Biofilmen auf Erlenlaub-Agar (ALA; n=8) oder supplementiertem Malz-Agar (SMA; n=8) Beschichtungen und unbeschichteten Objektträgern (UC; n=8). Kleine Buchstaben über den Kästchen zeigen signifikante Unterschiede an (Mann-Whitney U-Test, α: 0,05). Boxplots: siehe Abbildung 2.

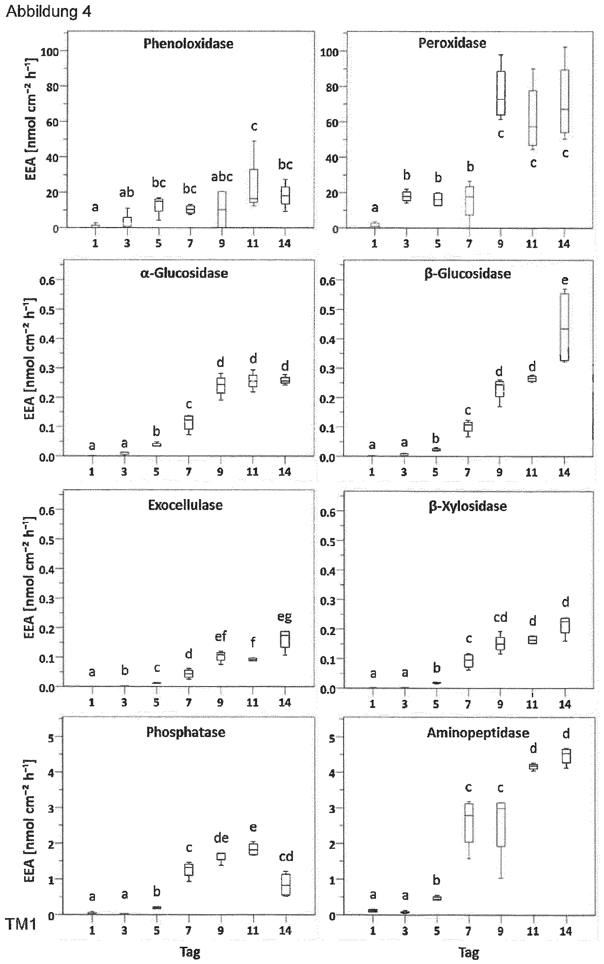

**Abb. 4** zeigt die Aktivitäten der extrazellulären Enzyme, gemessen in den Biofilmen an den Stichprobentagen 1-14 (n=4 für jeden Zeitschritt). Kleine Buchstaben über den Kästchen zeigen signifikante Unterschiede an (Mann-Whitney U-Test, p< 0,05). Boxplots: siehe Abbildung 2.

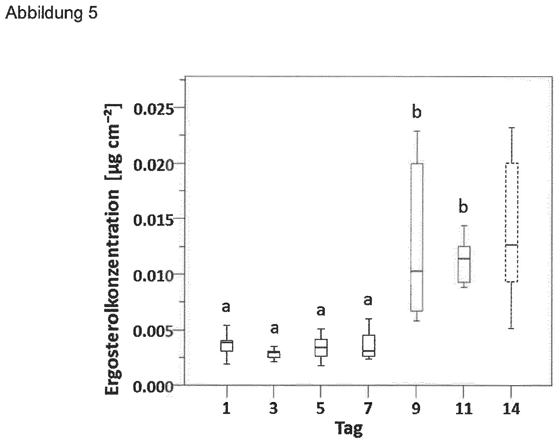

**Abb. 5** zeigt die Ergosterolkonzentrationen zwischen den Biofilmen der Probenahmetage 1-14 (Tag 1, 3, 7 und 11: n=4, Tag 9: n=6, Tag 14: n=7). (Die gestrichelte Box für die Daten von Tag 14 präsentiert Rohdaten, da die zur Berechnung der Extraktionseffizienz benötigten Referenzergosterolprobe während der Extraktions verloren ging.) Kleine Buchstaben über den Kästchen zeigen signifikante Unterschiede an (Mann-Whitney U-Test, α: 0,05). Boxplots: siehe Abbildung 2.

**Abb. 6** zeigt die Möglichkeiten zur Ausweitung des Verfahrens auf.

## Patentansprüche

1. Verfahren zur ökotoxikologischen Bewertung von mindestens einem Wirkstoff hinsichtlich der Auswirkungen auf einen Biofilm aus aquatischen Pilzen umfassend die folgenden Schritte:
i) Bereitstellung eines Trägers mit künstlichem Substrat, wobei das Substrat Erlenlaub-Agar umfassend 4% (Gew./Vol.) getrocknete, zerkleinerte Erlenblätter und 4% Agarose umfasst.
ii) In Kontaktbringung des Trägers mit dem künstlichen Substrat mit Wasser, welches ein Sporeninokulum aquatischer Pilze aufweist, so dass sich ein Biofilm aus aquatischen Pilzen auf dem Träger mit dem künstlichen Substrat bilden kann.
iii) Zugabe des mindestens einen zu testenden Wirkstoffs in das Wasser gemäß Schritt ii).
iv) Bestimmung der Biomasse aus aquatischen Pilzen im Biofilm aus Schritt ii).
v) Bestimmung des Stoffumsatzes der aquatischen Pilze im Biofilm aus Schritt ii) wobei der Einfluss mindestens eines Wirkstoffs auf die Werte aus Schritt iv) und/oder aus Schritt v) des Biofilms im Vergleich zu einer Vergleichsprobe erfasst werden, wobei die Vergleichsprobe anstatt des Wirkstoffes die gleiche Menge z.B. filtriertes und autoklaviertes Wasser oder bekannte Referenzsubstanzen aufweist.

2. Verfahren gemäß Anspruch 1 **dadurch gekennzeichnet, dass** der Träger ein Objektträger aus Glas ist.

3. Verfahren gemäß Anspruch 2 **dadurch gekennzeichnet, dass** der Objektträger wenigstens an der dem künstlichem Substrat zugewandten Seite aufgeraut oder angeschliffen ist.

4. Verfahren gemäß einem der vorangegangenen Ansprüche **dadurch gekennzeichnet, dass** die Kontaktbringung des Trägers mit dem künstlichen Substrat mit Wasser erfolgt, welches ein natürliches Sporeninokulum aus Referenzgewässern aufweist.

5. Verfahren gemäß einem der vorangegangenen Ansprüche **dadurch gekennzeichnet, dass** die Kontaktbringung des Trägers mit dem künstlichen Substrat mit Wasser erfolgt, welches ein gezieltes Sporeninokulum mit einer oder mehreren Pilzspezies aus Reinkulturen umfasst.

6. Verfahren gemäß einem der vorangegangenen Ansprüche **dadurch gekennzeichnet, dass** die Kontaktbringung des Trägers mit dem künstlichen Substrat mit Wasser innerhalb von Fließrinnen, Mesokosmen oder in Gewässern erfolgt.

7. Verfahren gemäß einem der vorangegangenen Ansprüche **dadurch gekennzeichnet, dass** die Kontaktbringung des Trägers mit dem künstlichen Substrat mit Wasser in Klimakammern mit standardisierbaren Bedingungen, Gebäuden mit seminatürlichen Bedingungen oder im Freiland erfolgt.

8. Verfahren gemäß einem der vorangegangenen Ansprüche **dadurch gekennzeichnet, dass** die Kontaktbringung des Trägers mit dem künstlichen Substrat und Wasser (inklusive Sporeninokulum) gemäß Schritt ii) innerhalb eines Inkubationszeitraumes der künstlichen Substrate von 9-14 Tagen in einem Biofilm mit ausreichender Biofilmmasse für ökotoxikologische Tests resultiert.

9. Verfahren gemäß einem der vorangegangenen Ansprüche **dadurch gekennzeichnet, dass** die Menge der Biomasse der aquatischen Pilzen des Biofilms in Schritt iv) durch die Messung der Ergosterol-Konzentration bestimmt wird.

10. Verfahren gemäß einem der vorangegangenen Ansprüche **dadurch gekennzeichnet, dass** die Bestimmung des Stoffumsatzes im Biofilm in Schritt v) durch Messung der extrazellulären Enzymaktivitäten von Phenoloxidase und Peroxidasen (pilzspezifische Enzymen), sowie α-Glukosidase, β-Glukosidase, Exocellulase, β-Xylosidase, Phosphatase und/oder Aminopeptidase erfolgt.

11. Verfahren gemäß einem der vorangegangenen Ansprüche **dadurch gekennzeichnet, dass** der mindestens eine Wirkstoff ein natürlicher oder chemischer Wirkstoff ist, insbesondere ausgewählt ist aus der Gruppe Pestizide, Fungizide, Herbizide, Insektizide, Medikamente, Antibiotika, Dünger, Schwermetalle, Salze, endokrine Disruptoren (hormonaktive Substanzen) und/oder Mikroplastik.

12. Verwendung eines Verfahrens gemäß einem der vorangegangenen Ansprüche zur Ermittlung von durch mindestens einen Wirkstoff hervorgerufene akute kurzfristige, kurzzeitige oder chronische langfristige langzeitige Wirkung auf aquatische Pilze in einem Gewässer.

## Claims

1. Method for the ecotoxicological evaluation of at least one active substance with regard to the effects on a biofilm of aquatic fungi comprising the following steps:
i) Providing a carrier comprising an artificial substrate, wherein the substrate comprises alder leaf agar comprising 4% (w/v) dried crushed alder leaves and 4% agarose.
ii) Bringing the carrier into contact with the artificial substrate with water containing a spore inoculum of aquatic fungi, so that a biofilm of aquatic fungi can form on the carrier with the artificial substrate.
iii) Addition of the at least one active substance to be tested to the water according to step ii).
iv) Determination of biomass from aquatic fungi in the biofilm from step ii).
v) Determination of the metabolic rate of the aquatic fungi in the biofilm from step ii) wherein the influence of at least one active substance on the values from step iv) and/or from step v) of the biofilm is recorded in comparison with a reference sample, wherein the reference sample comprises the same amount of e.g. filtered and autoclaved water or known reference substances instead of the active substance.

2. Method according to claim 1, **characterised in that** the support is a glass slide.

3. Method according to claim 2, **characterised in that** the slide is roughened or abraded at least on the side facing the artificial substrate.

4. Method according to one of the preceding claims, **characterised in that** the carrier is brought into contact with the artificial substrate with water containing a natural spore inoculum from reference waters.

5. Method according to one of the preceding claims, **characterised in that** the carrier is brought into contact with the artificial substrate with water comprising a targeted spore inoculum with one or more fungal species from pure cultures.

6. Method according to one of the preceding claims, **characterised in that** the carrier with the artificial substrate is brought into contact with water within flow channels, mesocosms or in bodies of water.

7. Method according to one of the preceding claims, **characterised in that** the carrier with the artificial substrate is brought into contact with water in climatic chambers with standardisable conditions, buildings with semi-natural conditions or in the open field.

8. Method according to one of the preceding claims, **characterised in that** bringing the carrier into contact with the artificial substrate and water (including spore inoculum) according to step ii) within an incubation period of the artificial substrates of 9-14 days results in a biofilm with sufficient biofilm mass for ecotoxicological tests.

9. Method according to one of the preceding claims, **characterised in that** the amount of biomass of the aquatic fungi of the biofilm is determined in step iv) by measuring the ergosterol concentration.

10. Method according to one of the preceding claims, **characterised in that** the determination of the metabolic rate in the biofilm in step v) is carried out by measuring the extracellular enzyme activities of phenol oxidase and peroxidases (fungus-specific enzymes), as well as α-glucosidase, β-glucosidase, exocellulase, β-xylosidase, phosphatase and/or aminopeptidase.

11. Method according to one of the preceding claims, **characterised in that** the at least one active substance is a natural or chemical active substance, in particular selected from the group consisting of pesticides, fungicides, herbicides, insecticides, medicaments, antibiotics, fertilisers, heavy metals, salts, endocrine disruptors (hormonally active substances) and/or microplastics.

12. Use of a method according to one of the preceding claims for determining acute short-term, short-term or chronic long-term effects on aquatic fungi in a body of water caused by at least one active substance.

## Revendications

1. Procédé d'évaluation écotoxicologique d'au moins une substance active en ce qui concerne les effets sur un biofilm de champignons aquatiques, comprenant les étapes suivantes:
i) Fourniture d'un support avec un substrat artificiel, ledit substrat comprenant de la gélose aux feuilles d'aulne comprenant 4% (poids/volume) de feuilles d'aulne séchées et broyées et 4% d'agarose.
ii) La mise en contact du support avec le substrat artificiel avec de l'eau contenant un inoculum de spores de champignons aquatiques, de sorte qu'un biofilm de champignons aquatiques puisse se former sur le support avec le substrat artificiel.
iii) Ajout d'au moins une substance active à tester dans l'eau selon l'étape ii).
iv) Détermination de la biomasse de champignons aquatiques dans le biofilm de l'étape ii).
v) Détermination du taux de conversion des champignons aquatiques dans le biofilm de l'étape ii)
l'influence d'au moins une substance active sur les valeurs de l'étape iv) et/ou de l'étape v) du biofilm étant saisie en comparaison avec un échantillon de comparaison, l'échantillon de comparaison présentant, au lieu de la substance active, la même quantité, par exemple d'eau filtrée et autoclavée ou de substances de référence connues.

2. Procédé selon la revendication 1, **caractérisé en ce que** le support est une lame de verre.

3. Procédé selon la revendication 2, **caractérisé en ce que** le porte-objet est rendu rugueux ou poncé au moins sur la face tournée vers le substrat artificiel.

4. Procédé selon l'une quelconque des revendications précédentes, **caractérisé en ce que** la mise en contact du support avec le substrat artificiel est effectuée avec de l'eau comprenant un inoculum de spores naturelles provenant d'eaux de référence.

5. Procédé selon l'une quelconque des revendications précédentes, **caractérisé en ce que** la mise en contact du support avec le substrat artificiel est effectuée avec de l'eau comprenant un inoculum de spores ciblé avec une ou plusieurs espèces fongiques de cultures pures.

6. Procédé selon l'une quelconque des revendications précédentes, **caractérisé en ce que** la mise en contact du support avec le substrat artificiel est effectuée avec de l'eau à l'intérieur de canaux d'écoulement, de mésocosmes ou dans des cours d'eau.

7. Procédé selon l'une quelconque des revendications précédentes, **caractérisé en ce que** la mise en contact du support avec le substrat artificiel est effectuée avec de l'eau dans des chambres climatiques aux conditions standardisables, des bâtiments aux conditions semi-naturelles ou en plein air.

8. Procédé selon l'une quelconque des revendications précédentes, **caractérisé en ce que** la mise en contact du support avec le substrat artificiel et l'eau (y compris l'inoculum de spores) selon l'étape ii) dans une période d'incubation des substrats artificiels de 9 à 14 jours résulte en un biofilm ayant une masse de biofilm suffisante pour des tests écotoxicologiques.

9. Procédé selon l'une des revendications précédentes, **caractérisé en ce que** la quantité de biomasse de champignons aquatiques du biofilm est déterminée à l'étape iv) par la mesure de la concentration d'ergostérol.

10. Procédé selon l'une des revendications précédentes **caractérisé en ce que** la détermination de la transformation de la substance dans le biofilm dans l'étape v) est effectuée par la mesure des activités enzymatiques extracellulaires de la phénoloxydase et des peroxydases (enzymes spécifiques aux champignons), ainsi que de l'α-glucosidase, de la β-glucosidase, de l'exocellulase, de la β-xylosidase, de la phosphatase et/ou de l'aminopeptidase.

11. Procédé selon l'une quelconque des revendications précédentes, **caractérisé en ce que** ladite au moins une substance active est une substance active naturelle ou chimique, notamment choisie dans le groupe constitué par les pesticides, les fongicides, les herbicides, les insecticides, les médicaments, les antibiotiques, les engrais, les métaux lourds, les sels, les perturbateurs endocriniens (perturbateurs hormonaux) et/ou les microplastiques.

12. Utilisation d'un procédé selon l'une des revendications précédentes pour déterminer l'effet aigu à court terme, à court terme ou chronique à long terme sur des champignons aquatiques dans un milieu aquatique, provoqué par au moins une substance active.
